# EUROPEAN PATENT APPLICATION

(11) **EP 3 628 657 A1**
(43) Date of publication of application: **01.04.2020**
(21) Application number: 18197724.0
(22) Date of filing: 28.09.2018
(51) Int. Cl.: C07C 209/60, C07C 211/08, C07C 211/27

(54) **PRODUCTION OF AMINES VIA A HYDROAMINOMETHYLATION REACTION USING IMINIUM REACTANTS**

(71) Applicant: Universität Wien, 1010 Vienna (AT)
(72) Inventor: MAULIDE, Nuno, 1090 Vienna (AT); TONA, Veronica, 1090 Vienna (AT)
(74) Representative: Vossius & Partner Patentanwälte Rechtsanwälte mbB

(57) **Abstract**

Provided is a process for producing an amine via a hydroaminomethylation reaction of a non-aromatic C-C double bond, said process comprising
a step of reacting a compound comprising a non-aromatic C-C double bond with an iminium ion in a solvent comprising an alcohol, wherein the iminium ion is represented by the following formula (I): 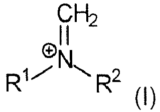 wherein R¹ and R² are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted; and R¹ and R² may be bonded to each other to form a ring together with the nitrogen atom to which they are bound; and wherein R¹ and R² are not both hydrogen and at least one of R¹ and R² carries a hydrogen atom at a carbon atom in α-position to the nitrogen atom of the iminium ion.

## Description

The present invention relates to a process for producing an amine via a hydroaminomethylation reaction of a non-aromatic C-C double bond.

Amines are key structural elements in our life, as they constitute core elements of a variety of biologically active agents and crucial high-performance materials. Accordingly, the synthesis of amino compounds from abundant and readily available substrates is of crucial importance to chemistry, biology, medicine and materials science. In particular, the synthesis of amines starting from substituted or non-substituted alkenes and alkynes is of relevance. A direct functionalization of such alkenes and alkynes can be achieved by a hydroaminomethylation reaction, addition of hydrogen and a methylamine-unit across an olefin.

State-of-the-art protocols for hydroaminomethylation of alkenes rely largely on transition-metal catalysis, which enables hydroaminomethylation under highly designed and controlled conditions. Several metal-catalyzed hydroaminomethylations of alkenes with amines have been reported in recent years. The intermolecular addition of the α-C-H bonds of a dialkylamine to an unactivated olefin in the presence of a chloro amido tantalum complex is described in "Hydroaminoalkylation of unactivated olefins with dialkylamines" of Herzon, S. B. & Hartwig, J. F., J. Am. Chem. Soc. 2008, 130, 14940-14941. A catalytic hydroaminomethylation of an alkene with dimethylamine using a titanium catalyst is described in "Dimethylamine as a substrate in hydroaminoalkylation reactions" of Bielefeld, J. & Doye, S., Angew. Chem. Int. Ed. 2017, 56, 15155-15158. While proceeding under catalytic conditions and employing simple amines as starting materials, metal-catalyzed hydroaminomethylation reactions, dominated by group 4 and group 5 metal catalysts, have shown some restrictions in terms of selectivity and functional group tolerance.

An alternative approach to the hydroaminomethylation of alkenes lies in photocatalysis or dual catalysis using transition-metal and photocatalysis. α-Aminoalkyl radicals have been added to electron-deficient alkenes by visible-light-mediated electron transfer using transition metal polypyridyl complexes as photocatalysts (Miyake, Y., Nakajima, K. & Nishibayashi, Y., "Visible-light-mediated utilization of α-aminoalkyl radicals: addition to electron-deficient alkenes using photoredox catalysts", J. Am. Chem. Soc. 2012, 134, 3338-3341). The coupling of photoredox-generated α-amino radical species with conjugated dienes using a unified cobalt and iridium catalytic system in order to access a variety of useful homoallylic amines from simple commercially available starting materials is described in "A mild hydroaminoalkylation of conjugated dienes using a unified cobalt and photoredox catalytic system" of Thullen, S. M. & Rovis, T., J. Am. Chem. Soc. 2017, 139, 15504-15508. Even though mild reaction conditions can be applied, photocatalytic hydroaminomethylation reactions are limited to specific substrates.

In order to address these challenges, the present inventors became interested in the development of a redox-neutral hydroaminomethylation reaction circumventing the need for early transition-metal catalysis and photocatalysis. In a hydroaminomethylation reaction of 2-phenyl-2-norbornene with formaldehyde and dimethylamine in acetic acid, a mixture of compounds best explained by a 1,5-hydride shift in one intermediate step is produced (Manninen, K. & Haapala, J., "The Reaction of 2-Phenyl-2-norbornene with Formaldehyde and Dimethylamine. Additional Evidence for the Occurrence of a 1,5-Hydride Shift during the Aminomethylation of a Strained Bicycloalkene Structure", Acta Chem. Scand. B, 1974, 28, 433-440). A hydroaminomethylation of 1-alkenes with an iminium ion obtained from Eschenmoser's salt or generated by the action of phosphoric or sulfuric acid on (tetramethyldiamino)methane in acetonitrile or acetic acid results in secondary amines and unsaturated tertiary amines (Cohen, T. & Onopchenko, A., "Competing Hydride Transfer and Ene Reactions in the Aminoalkylation of 1-Alkenes with N,N-Dimethyleniminium Ions. A Literature Correction", J. Org. Chem., 1983, 48, 4531-4537). However, also known redox-neutral hydroaminomethylation reactions suffer from certain limiations, such as formation of elimination products and other side products, low yields and limited functional group tolerance.

In the context of the present invention, it has been found that the use of an iminium ion in an alcohol solvent in a hydroaminomethylation reaction of non-aromatic C-C double bond can markedly influence this reaction in that competing elimination reactions are suppressed and high product yields, a broad functional group tolerance and linear selectivities are achieved. A broadly applicable, redox-neutral approach to hydroaminomethylation of compounds comprising a non-aromatic C-C double bondis thus made available in order to obtain secondary amines. Due to the mild conditions of the reported hydroaminomethylation reaction further functionalization of the reaction intermediates is possible. Thus, for example, tertiary amines can be conveniently obtained.

The present invention provides a process for producing an amine via a hydroaminomethylation reaction of a non-aromatic C-C double bond, said process comprising a step of reacting a compound comprising a non-aromatic C-C double bond with an iminium ion in a solvent comprising an alcohol,
wherein the iminium ion is represented by the following formula (I): 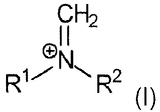 wherein,
R¹ and R² are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted;
and R¹ and R² may be bonded to each other to form a ring together with the nitrogen atom to which they are bound;
and wherein R¹ and R² are not both hydrogen and at least one of R¹ and R² carries a hydrogen atom at a carbon atom in α-position to the nitrogen atom of the iminium ion.

An exemplary hydroaminomethylation reaction of a non-aromatic C-C double bond in accordance with the present invention is illustrated by the following reaction scheme 1.

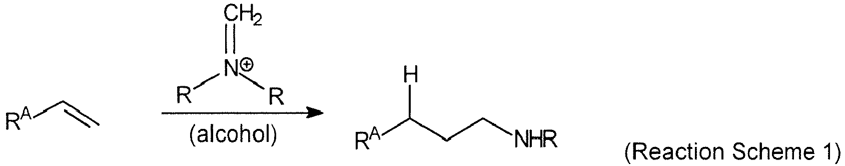

In this reaction, a non-aromatic C-C double bond is converted with an iminium ion in a solvent comprising an alcohol to an amine. The groups R and R^{A} represent any desired atoms or groups, and it should be understood that the compound comprising a non-aromatic C-C double bond and the iminium ion used in the process of the present invention are not limited to the exemplified structures as shown in this scheme.

As noted above, the process of the present invention encompasses a hydroaminomethylation reaction of a non-aromatic C-C double bond. Thus, it comprises a step of reacting a compound comprising a non-aromatic C-C double bond with an iminium ion in a solvent comprising an alcohol.

### Iminium ion

In the process in accordance with the present invention, a compound comprising a non-aromatic C-C double bond is reacted with an iminium ion in a solvent comprising an alcohol.

The iminium ion used in the process in accordance with the present invention is represented by the following formula (I): 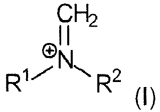 wherein R¹ and R² are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted, wherein R¹ and R² may be bonded to each other to form a ring together with the nitrogen atom indicated in formula (I) to which R¹ and R² are bound. R¹ and R² are not both hydrogen simultaneously. Moreover, at least one of R¹ and R² carries a hydrogen atom at a carbon atom in α-position to the nitrogen atom indicated in formula (I) for the iminium ion.

As noted above, at least one of R¹ and R² of the iminium ion of formula (I) carries a hydrogen atom at a carbon atom bound in α-position to the nitrogen atom. As will be understood by the skilled reader, a carbon atom bound in α-position to the nitrogen atom is a carbon atom (generally a sp³-hybridized carbon atom) which is directly bound to the nitrogen atom. Thus, the iminium ion contains a structural unit which may be represented as

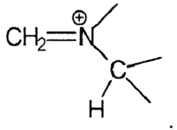

In formula (I), R¹ and R² are preferably independently selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted, and wherein R¹ and R² may be bonded to each other to form a ring together with the nitrogen atom to which they are bound. More preferably, R¹ and R² are an aliphatic hydrocarbon group which may be substituted.

It is noted that, in the formulae described herein, any hydrogen atom can generally be replaced by deuterium. This applies not only for formula (I) above, but also to formula (II) below.

The aliphatic hydrocarbon group has preferably 1 to 30 carbon atoms, more preferably 1 to 24 carbon atoms, and even more preferably 1 to 12 carbon atoms. The aromatic hydrocarbon group has preferably 6 to 30 carbon atoms, more preferably 6 to 22 carbon atoms, and even more preferably 6 to 14 carbon atoms. The aliphatic heterocyclic group has preferably 2 to 30 carbon atoms, more preferably 2 to 24 carbon atoms, and even more preferably 2 to 12 carbon atoms. The aromatic heterocyclic group has preferably 2 to 30 carbon atoms, more preferably 3 to 22 carbon atoms, and even more preferably 3 to 12 carbon atoms. The aralkyl group has preferably 7 to 30 carbon atoms, more preferably 7 to 24 carbon atoms, and even more preferably 7 to 14 carbon atoms.

The aliphatic heterocyclic group and the aromatic heterocyclic group have independently preferably 1 to 5 heteroatoms, more preferably 1 to 3 heteroatoms. The heteroatom is preferably selected from the group consisting of a nitrogen atom, an oxygen atom and a sulphur atom.

The aliphatic hydrocarbon group, the aromatic hydrocarbon group, the aliphatic heterocyclic group, the aromatic heterocyclic group, alone or in combination, may be substituted with one or more, such as one, two or three, substituents. Exemplary substituents for the mentioned groups are an aliphatic hydrocarbon group having 1 to 12 carbon atoms, an aromatic hydrocarbon group having 6 to 14 carbon atoms, an aliphatic heterocyclic group having 2 to 12 carbon atoms and 1 to 3 heteroatoms, an aromatic heterocyclic group having 3 to 12 carbon atoms and 1 to 3 heteroatoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may in turn carry one or more, such as one, two or three, substituents selected from an aliphatic hydrocarbon group having 1 to 12 carbon atoms, an aromatic hydrocarbon group having 6 to 14 carbon atoms, an aliphatic heterocyclic group having 2 to 12 carbon atoms and 1 to 3 heteroatoms, an aromatic heterocyclic group having 3 to 12 carbon atoms and 1 to 3 heteroatoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

Suitable examples of an aliphatic hydrocarbon group are a linear or branched alkyl group, a cycloalkyl group, a linear or branched alkenyl group, a cycloalkenyl group and a linear or branched alkynyl group. A preferred example of an aliphatic hydrocarbon group is a linear alkyl group. Suitable examples of an aliphatic heterocyclic group are a heterocycloalkyl group and a heterocycloalkenyl group. The aromatic heterocyclic group may also be referred to as a heteroaromatic group.

A linear alkyl group as referred to herein has preferably 1 to 30 carbon atoms, more preferably 1 to 24 carbon atoms, even more preferably 1 to 12 carbon atoms and most preferably 1 to 6 carbon atoms. A branched alkyl group as referred to herein has preferably 3 to 30 carbon atoms, more preferably 3 to 24 carbon atoms, even more preferably 3 to 12 carbon atoms and most preferably 3 to 6 carbon atoms. A cycloalkyl group as referred to herein has preferably 3 to 30 carbon atoms, more preferably 3 to 24 carbon atoms, and even more preferably 3 to 12 carbon atoms. A linear alkenyl group as referred to herein has preferably 2 to 30 carbon atoms, more preferably 2 to 24 carbon atoms, and even more preferably 2 to 12 carbon atoms. A branched alkenyl group as referred to herein has preferably 4 to 30 carbon atoms, more preferably 4 to 24 carbon atoms, and even more preferably 4 to 12 carbon atoms. A cycloalkenyl group as referred to herein has preferably 3 to 30 carbon atoms, more preferably 3 to 24 carbon atoms, and even more preferably 3 to 12 carbon atoms. A linear alkynyl group as referred to herein has preferably 2 to 30 carbon atoms, more preferably 2 to 24 carbon atoms, and even more preferably 2 to 12 carbon atoms. A branched alkynyl group as referred to herein has preferably 4 to 30 carbon atoms, more preferably 4 to 24 carbon atoms, and even more preferably 4 to 12 carbon atoms. An aromatic hydrocarbon group as referred to herein has preferably 6 to 30 carbon atoms, more preferably 6 to 22 carbon atoms, and even more preferably 6 to 14 carbon atoms. A heterocycloalkyl group as referred to herein has preferably 2 to 30 carbon atoms and 1 to 5 heteroatoms, more preferably 2 to 24 carbon atoms and 1 to 5 heteroatoms, and even more preferably 2 to 12 carbon atoms and 1 to 3 heteroatoms. A heterocycloalkenyl group as referred to herein has preferably 2 to 30 carbon atoms and 1 to 5 heteroatoms, more preferably 2 to 24 carbon atoms and 1 to 5 heteroatoms, and even more preferably 2 to 12 carbon atoms and 1 to 3 heteroatoms. A heteroaromatic hydrocarbon group as referred to herein has preferably 2 to 30 carbon atoms and 1 to 5 heteroatoms, more preferably 3 to 22 carbon atoms and 1 to 5 heteroatoms, and even more preferably 3 to 12 carbon atoms and 1 to 3 heteroatoms. The heteroatom is preferably selected from the group consisting of a nitrogen atom, an oxygen atom and a sulphur atom.

In formula (I), R¹ and R² are more preferably independently selected from the group consisting of a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted, and R¹ and R² may be bonded to each other to form a ring together with the nitrogen atom to which they are bound.

Also with these preferred groups R¹ and R², at least one of R¹ and R² carries a hydrogen atom at a carbon atom in α-position to the nitrogen atom, and preferably R¹ and R² are identical, i.e. R¹ and R² have the same structure.

The optionally substituted groups may be substituted by one or more, such as one, two or three, substituents. Also with these preferred groups R¹ and R², exemplary substituents for the optionally substituted groups are selected from an alkyl group having 1 to 12 carbon atoms, a cycloalkyl group having 3 to 12 carbon atoms, a heterocycloalkyl group having 2 to 12 carbon atoms, an alkenyl group having 2 to 12 carbon atoms, a cycloalkenyl group having 3 to 12 carbon atoms, an alkynyl group having 2 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may carry in turn one or more, such as one, two or three, substituents selected from an alkyl group having 1 to 12 carbon atoms, a cycloalkyl group having 3 to 12 carbon atoms, a heterocycloalkyl group having 2 to 12 carbon atoms, an alkenyl group having 2 to 12 carbon atoms, a cycloalkenyl group having 3 to 12 carbon atoms, an alkynyl group having 2 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

In formula (I), R¹ and R² are even more preferably independently selected from the group consisting of methyl, ethyl, propyl, isopropyl, butyl, isobutyl, benzyl and allyl. Also these even more preferred groups R¹ and R² are preferably identical, i.e. R¹ and R² have the same structure.

The iminium ion used in accordance with the present invention is most preferably dimethylmethylideneammonium.

The iminium ion used in the process according to the present invention is preferably in the form of an iminium salt. The iminium salt includes an anion. The anion is not particulary limited and is preferably a monovalent anion. Examples of the anion include iodide, bromide, chloride, nitrate, triflate and trifluoroacetate.

Preferred examples of the iminium salt for use in the process according to the present invention include dimethylmethylideneammonium iodide, dimethylmethylideneammonium chloride and dimethylmethylideneammonium nitrate, more preferably dimethylmethylideneammonium iodide (i.e. Eschenmoser's salt).

Iminium salts suitable for use in the process of the present invention are commercially available, or can be prepared by synthetic methods e.g. as reported in the work of Eschenmoser (Angew. Chem. Int. Ed. 1971, 10, 330 - 331). A two step synthesis from secondary amines can also be employed, where the amine is first converted to the aminoether via condensation with paraformaldehyde in ethanol (Tryptamines as Ligands and Modulators of the Serotonin 5-HT2A Receptor and the Isolation of Aeruginascin from the Hallucinogenic Mushroom Inocybe aeruginascens - Disseration of Niels Jensen, https://d-nb.info/973960833/34) then the ether is treated with trimethylchlorosilane to give the iminium chloride (Rochin, C., Babot, O., Dunoguès, J., Duboudin, F. Synthesis 1986, 3, 228-229. DOI: 10.1055/s-1986-31627). Alternatively, trimethyliodosilane can be used to obtain the iminium iodide.

### Compound comprising a non-aromatic C-C double bond

According to the present invention, a compound comprising a non-aromatic C-C double bond is reacted with the iminium ion in a solvent comprising an alcohol.

The compound comprising a non-aromatic C-C double bond (i.e. a non-aromatic double bond which links two carbon atoms) which is subjected to the reaction with the iminium ion in accordance with the present invention is an organic compound which may comprise one non-aromatic C-C double bond or multiple, i.e. two or more, non-aromatic C-C double bonds. If the compound comprises multiple C-C double bonds, the double bonds may be conjugated double bonds. In a case of multiple C-C double bonds, the process in accordance with the invention may be applicable for multiple hydroaminomethylation reactions in a single compound.

Preferably, the compound comprising a non-aromatic C-C double bond comprises one or two non-aromatic C-C double bonds, more preferably one non-aromatic C-C double bond.

Since the non-aromatic C-C double bond represents the relevant functional feature of the compound subjected to the hydroaminomethylation reaction, the compound comprising the non-aromatic C-C double bond may also be referred to as an "alkene" herein. Unless indicated otherwise, this reference to an alkene includes the optional presence of optional subsitituents bound to the hydrocarbon core structure of the alkene.

The structure of the compound comprising a non-aromatic C-C double bond is not particularly limited. For example, the non-aromatic C-C double bond may form part of a linear, a branched or a ring structure, or of a linear, a branched or a ring substructure in the compound comprising the non-aromatic C-C double bond. In addition to the non-aromatic C-C double bond, the compound may comprise one or more additional functional groups and/or aromatic double bonds and/or a C-C triple bond.

Preferably, the non-aromatic C-C double bond (or the non-aromatic C-C double bonds, if multiple double bonds are present) is/are selected from a non-substituted, a monosubstituted, a disubstituted and a trisubstituted non-aromatic C-C double bond. More preferably, the non-aromatic C-C double bond (or the non-aromatic C-C double bonds, if multiple double bonds are present) is/are selected from a monosubstited and a disubstituted non-aromatic C-C double bond. As will be understood by the skilled reader, a non-substituted C-C double bond is a C-C double bond wherein neither one of the two carbon atoms linked by the double bond carries a substituent other than a hydrogen (i.e. the compound comprising the non-substituted C-C double bond is ethene). In a monsubstituted C-C double bond, one of the carbon atoms linked by the double bond carries one substituent other than hydrogen. Reference to a disubstituted C-C double bond herein refers, unless specifically indicated otherwise, to a C-C double bond wherein each of the two carbon atoms linked by the double bond carries one substituent other than a hydrogen atom. Accordingly, in a trisubstituted C-C double bond, one of the carbon atoms linked by the double bond carries two substituents other than hydrogen, the other one carries one substituent other than hydrogen. Preferably, the substituents other than hydrogen are substituted or non-substituted hydrocarbon groups.

A preferred, exemplary compound comprising a non-aromatic C-C double bond for use in the process in accordance with the present invention is represented by the following formula (II): 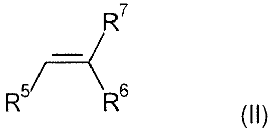 wherein R⁵, R⁶ and R⁷ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted, and of combinations thereof, such as an aralkyl group which may be substituted, and wherein R⁵ and R⁶ may be bonded to each other to form a ring together with the carbon atoms to which they are bound.

The aliphatic hydrocarbon group has preferably 1 to 30 carbon atoms, more preferably 1 to 24 carbon atoms, and even more preferably 1 to 12 carbon atoms. The aromatic hydrocarbon group has preferably 6 to 30 carbon atoms, more preferably 6 to 22 carbon atoms, and even more preferably 6 to 14 carbon atoms. The aliphatic heterocyclic group has preferably 2 to 30 carbon atoms, more preferably 2 to 24 carbon atoms, and even more preferably 2 to 12 carbon atoms. The aromatic heterocyclic group has preferably 2 to 30 carbon atoms, more preferably 3 to 22 carbon atoms, and even more preferably 3 to 12 carbon atoms. The aralkyl group has preferably 7 to 30 carbon atoms, more preferably 7 to 24 carbon atoms, and even more preferably 7 to 14 carbon atoms.

The aliphatic heterocyclic group and the aromatic heterocyclic group contain independently preferably 1 to 5 heteroatoms, more preferably 1 to 3 heteroatoms. The heteroatom is preferably selected from a nitrogen atom, an oxygen atom and a sulphur atom.

The aliphatic hydrocarbon group, the aromatic hydrocarbon group, the aliphatic heterocyclic group, the aromatic heterocyclic group, alone or in combination, may be substituted by one or more, such as one two or three, substituents. Exemplary substituents are aliphatic hydrocarbon groups having 1 to 12 carbon atoms, aromatic hydrocarbon groups having 6 to 14 carbon atoms, aliphatic heterocyclic groups having 2 to 12 carbon atoms and 1 to 3 heteroatoms, aromatic heterocyclic groups having 3 to 12 carbon atoms and 1 to 3 heteroatoms, halogen atoms, oxo groups, hydroxyl groups, cyano groups, amino groups, carbamoyl groups, carboxyl groups, alkoxy groups having 1 to 10 carbon atoms, alkoxycarbonyl groups having 2 to 10 carbon atoms, alkanoyloxy groups having 2 to 10 carbon atoms, phosphonate groups and trialkylsilyl groups having 3 to 30 carbon atoms. Where suitable, these exemplary substituents may in turn be substituted by one or more, such as one, two or three, substituents selected from aliphatic hydrocarbon groups having 1 to 12 carbon atoms, aromatic hydrocarbon groups having 5 to 14 carbon atoms, aliphatic heterocyclic groups having 2 to 12 carbon atoms and 1 to 3 heteroatoms, aromatic heterocyclic groups having 3 to 12 carbon atoms and 1 to 3 heteroatoms, halogen atoms, oxo groups, hydroxyl groups, cyano groups, amino groups, carbamoyl groups, carboxyl groups, alkoxy groups having 1 to 10 carbon atoms, alkoxycarbonyl groups having 2 to 10 carbon atoms, alkanoyloxy groups having 2 to 10 carbon atoms, phosphonate groups and trialkylsilyl groups having 3 to 30 carbon atoms.

Suitable examples of an aliphatic hydrocarbon group are a linear or branched alkyl group, a cycloalkyl group, a linear or branched alkenyl group, a cycloalkenyl group and a linear or branched alkynyl group. Suitable examples of an aliphatic heterocyclic group are a heterocycloalkyl group and a heterocycloalkenyl group. The aromatic heterocyclic group may also be referred to as a heteroaromatic group.

A linear alkyl group as referred to herein has preferably 1 to 30 carbon atoms, more preferably 1 to 24 carbon atoms, and even more preferably 1 to 12 carbon atoms. A branched alkyl group as referred to herein has preferably 3 to 30 carbon atoms, more preferably 3 to 24 carbon atoms, and even more preferably 3 to 12 carbon atoms. A cycloalkyl group as referred to herein has preferably 3 to 30 carbon atoms, more preferably 3 to 24 carbon atoms, and even more preferably 3 to 12 carbon atoms. A linear alkenyl group as referred to herein has preferably 2 to 30 carbon atoms, more preferably 2 to 24 carbon atoms, and even more preferably 2 to 12 carbon atoms. A branched alkenyl group as referred to herein has preferably 4 to 30 carbon atoms, more preferably 4 to 24 carbon atoms, and even more preferably 4 to 12 carbon atoms. A cycloalkenyl group as referred to herein has preferably 3 to 30 carbon atoms, more preferably 3 to 24 carbon atoms, and even more preferably 3 to 12 carbon atoms. A linear alkynyl group as referred to herein has preferably 2 to 30 carbon atoms, more preferably 2 to 24 carbon atoms, and even more preferably 2 to 12 carbon atoms. A branched alkynyl group as referred to herein has preferably 4 to 30 carbon atoms, more preferably 4 to 24 carbon atoms, and even more preferably 4 to 12 carbon atoms. An aromatic hydrocarbon group as referred to herein has preferably 6 to 30 carbon atoms, more preferably 6 to 22 carbon atoms, and even more preferably 6 to 14 carbon atoms. A heterocycloalkyl group as referred to herein has preferably 2 to 30 carbon atoms and 1 to 5 heteroatoms, more preferably 2 to 24 carbon atoms and 1 to 5 heteroatoms, and even more preferably 2 to 12 carbon atoms and 1 to 3 heteroatoms. A heterocycloalkenyl group as referred to herein has preferably 2 to 30 carbon atoms and 1 to 5 heteroatoms, more preferably 2 to 24 carbon atoms and 1 to 5 heteroatoms, and even more preferably 2 to 12 carbon atoms and 1 to 3 heteroatoms. A heteroaromatic hydrocarbon group as referred to herein has preferably 2 to 30 carbon atoms and 1 to 5 heteroatoms, more preferably 3 to 22 carbon atoms and 1 to 5 heteroatoms, and even more preferably 3 to 12 carbon atoms and 1 to 3 heteroatoms. The heteroatom is preferably selected from the group consisting of a nitrogen atom, an oxygen atom and a sulphur atom.

In formula (II), R⁵, R⁶ and R⁷ are preferably independently selected from the group consisting of hydrogen, a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted, and R⁵ and R⁶ may be bonded to each other to form a ring together with the carbon atoms to which they are bound.

The optionally substituted groups may be substituted by one or more, such as one, two or three, substituents. Exemplary substituents for the optionally substituted groups are selected from an alkyl group having 1 to 12 carbon atoms, a cycloalkyl group having 3 to 12 carbon atoms, a heterocycloalkyl group having 2 to 12 carbon atoms, an alkenyl group having 2 to 12 carbon atoms, a cycloalkenyl group having 3 to 12 carbon atoms, an alkynyl group having 2 to 12 carbon atoms, an aromatic hydrocarbon group having 6 to 14 carbon atoms, a heteroaromatic group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may in turn carry one or more, such as one, two or three, substituents selected from an alkyl group having 1 to 12 carbon atoms, a cycloalkyl group having 3 to 12 carbon atoms, a heterocycloalkyl group having 2 to 12 carbon atoms, an alkenyl group having 2 to 12 carbon atoms, a cycloalkenyl group having 3 to 12 carbon atoms, an alkynyl group having 2 to 12 carbon atoms, an aromatic hydrocarbon group having 6 to 14 carbon atoms, a heteroaromatic group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

In formula (II), more preferably R⁵ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic hydrocarbon group which may be substituted; and either
(i) R⁶ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic group which may be substituted; R⁵ and R⁶ may be bonded to each other to form a ring together with the carbon atoms to which they are bound; and R⁷ is selected from the group consisting of hydrogen and methyl, and is more preferably hydrogen; or
(ii) R⁷ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic group which may be substituted; and R⁶ is selected from the group consisting of hydrogen and methyl, and is more preferably hydrogen.

Also in this case, the optionally substituted groups may be substituted by one or more, such as one, two or three, substituents. Exemplary substituents for the optionally substituted groups are selected from an alkyl group having 1 to 12 carbon atoms, a cycloalkyl group having 3 to 12 carbon atoms, a heterocycloalkyl group having 2 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may in turn carry one or more, such as one, two or three, substituents selected from an alkyl group having 1 to 12 carbon atoms, a cycloalkyl group having 3 to 12 carbon atoms, a heterocycloalkyl group having 2 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

In formula (II), even more preferably R⁵ is selected from the group consisting of hydrogen, an alkyl group which may be substituted, a cycloalkyl group which may be substituted; and either
(i) R⁶ is selected from the group consisting of hydrogen, an alkyl group which may be substituted, a cycloalkyl group which may be substituted, an aromatic hydrocarbon group which may be substituted and an aralkyl group which may be substituted; and R⁵ and R⁶ may be bonded to each other to form a ring together with the carbon atoms to which they are bound; and R⁷ is selected from the group consisting of hydrogen and methyl, and is still more preferably hydrogen; or
(ii) R⁷ is selected from the group consisting of hydrogen, an alkyl group which may be substituted, a cycloalkyl group which may be substituted, an aromatic hydrocarbon group which may be substituted and an aralkyl group which may be substituted; and R⁶ is selected from the group consisting of hydrogen and methyl, and is still more preferably hydrogen.

The optionally substituted groups may be substituted by one or more, such as one, two or three, substituents. Exemplary substituents for the optionally substituted groups are selected from an alkyl group having 1 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms. Where suitable, these exemplary substituents may in turn carry one or more, such as one, two or three, substituents selected from an alkyl group having 1 to 12 carbon atoms, an aromatic hydrocarbon group having 5 to 14 carbon atoms, a heteroaromatic hydrocarbon group having 3 to 12 carbon atoms, a halogen atom, an oxo group, a hydroxyl group, a cyano group, an amino group, a carbamoyl group, a carboxyl group, an alkoxy group having 1 to 10 carbon atoms, an alkoxycarbonyl group having 2 to 10 carbon atoms, an alkanoyloxy group having 2 to 10 carbon atoms, a phosphonate group and a trialkylsilyl group having 3 to 30 carbon atoms.

### Solvent comprising an alcohol

In the process of the present invention, the compound comprising a non-aromatic C-C double bond is reacted with the iminium ion in a solvent comprising an alcohol.

The present inventors surprisingly found that the use of a solvent comprising an alcohol in the hydroaminomethylation reaction allows high yields and a high selectivity of the amine to be obtained.

The solvent used in accordance with the present invention comprises an alcohol or consists of an alcohol. In a solvent comprising an alcohol, a further solvent other than an alcohol may be contained; thus, a combination of an alcohol and a further solvent other than an alcohol may be used as a solvent. Such combinations typically comprise more than 50 vol%, preferably more than 70 vol% of the alcohol, based on the total volume of solvent. Most preferably, the solvent consists of an alcohol.

As will be understood by the skilled reader, an alcohol for use in the process of the present invention is an organic compound comprising one or more hydroxy groups, preferably one hydroxy group, which hydroxy group(s) is/are attached to a carbon atom. The alcohol suitable for use as a solvent is generally liquid under standard conditions (i.e. 25°C and 100kPa). The alcohol for use in the process in accordance with the invention preferably has one to seven carbon atoms, more preferably two to seven carbon atoms. Thus, it is preferred to use an alcohol comprising two to seven carbon atoms, and one hydroxy group attached to one of the carbon atoms.

The alcohol is preferably a halogenated alcohol, i.e. an alcohol which carries at least one halogen substitutent, such as a fluorine atom, a chlorine atom or a bromine atom, in addition to the hydroxy group(s). Preferably, the halogenated alcohol is a fluorinated alcohol which carries at least one fluorine substitutent in addition to the hydroxy group(s).

In accordance with very preferred embodiements, the solvent wherein the reaction is carried out comprises or consists of a fluorinated alcohol selected from 1-fluoroethanol; 2-fluoroethanol; trifluoroethanol; 3,3,3-trifluoro-1-propanol; 1,1,1-trifluoro-2-propanol; 2,2,3,3,3-pentafluoro-1-propanol; hexafluoroisopropanol; 1,1,1-trifluoro-2-butanol; 4,4,4-trifluoro-1-butanol; 1,1,1-trifluoro-3-methyl-2-butanol; 4,4,5,5,5-pentafluoro-1-pentanol; 3-fluorophenethyl alcohol; 2,4,5-trifluorobenzyl alcohol; 2- fluorophenol; 3- fluorophenol; 4-fluorophenol and from combinations thereof. More preferably, the solvent wherein the reaction is carried out comprises or consists of a fluorinated alcohol selected from 1-fluoroethanol; 2-fluoroethanol; trifluoroethanol; hexafluoroisopropanol; 2- fluorophenol; 3-fluorophenol; 4-fluorophenol and from combinations thereof. Still more preferably, the solvent wherein the reaction is carried out comprises or consists of a fluorinated alcohol selected from trifluoroethanol; hexafluoroisopropanol and from combinations thereof; most preferably the solvent comprises or consists of hexafluoroisopropanol.

As described above, in the solvent comprising an alcohol, the alcohol may be present in combination with a further solvent other than an alcohol. Examples of the further solvent other than an alcohol include benzene, substituted benzene such as chlorbenzene or toluene, dichloroethene (DCE), ester solvents such as ethyl acetate, dimethylformamide (DMF), dimethylsulfoxide (DMSO), chloroform, and dichloromethane (DCM).

In line with the above, it will be understood that it is generally preferred for the process of the present invention if the iminium ion is dimethylmethylideneammonium, and the solvent comprises or consists of a fluorinated alcohol, more preferably a fluorinated alcohol selected from 1-fluoroethanol, 2-fluoroethanol, trifluoroethanol, hexafluoroisopropanol, 2-fluorophenol, 3- fluorophenol, and 4-fluorophenol and from combinations thereof, more preferably from hexafluoroisopropanol and trifluoroethanol, and combinations thereof, and most preferably the alcohol comprises or consists of hexafluoroisopropanol.

### Hydroaminomethylation Reaction and further process steps

In the process of the present invention, the compound comprising a non-aromatic C-C double bond is reacted with the iminium ion in the solvent comprising an alcohol. All the reactants and their preferred embodiments are described in detail above.

The process in accordance with the invention may further comprise a step, wherein the iminium ion being in the form of an iminium salt is combined with the compound comprising a non-aromatic C-C double bond in the solvent comprising an alcohol.

Such a preferred process in accordance with the invention is therefore defined as a process for producing an amine via a hydroaminomethylation reaction of a non-aromatic C-C double bond, said process comprising
a step of combining an iminium ion in the form of an iminium salt with a compound comprising a non-aromatic C-C double bond in a solvent comprising an alcohol, and
a step of reacting the compound comprising a non-aromatic C-C double bond with the iminium ion in a solvent comprising an alcohol,
wherein the iminium ion is represented by the following formula (I): 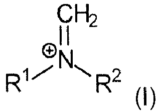 wherein R¹ and R² are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted; and R¹ and R² may be bonded to each other to form a ring together with the nitrogen atom to which they are bound; and wherein R¹ and R² are not both hydrogen and at least one of R¹ and R² carries a hydrogen atom at a carbon atom in α-position to the nitrogen atom of the iminium ion.

Typically, the iminium ion is combined with the compound comprising a non-aromatic C-C double bond simply by mixing the iminium ion and the compound comprising a non-aromatic C-C double bond in a solvent comprising an alcohol, with at least the iminium ion being in the form of an iminium salt.

The iminium ion may be combined with the compound comprising a non-aromatic C-C double bond e.g. by first providing the iminium ion in a reaction vessel, and adding the compound comprising a non-aromatic C-C double bond to the iminium ion, or by first providing the compound comprising a non-aromatic C-C double bond as a first component in a reaction vessel, and adding the iminium ion to the compound comprising a non-aromatic C-C double bond.

The combination of the iminium ion with the compound comprising a non-aromatic C-C double bond and the solvent comprising an alcohol may be accomplished in multiple steps, e.g. by first combining the iminium ion with the compound comprising a non-aromatic C-C double bond, and subsequently combining the mixture with the solvent comprising an alcohol.

Preferably, the iminium ion is first provided in the reaction vessel and then the compound comprising a non-aromatic C-C double bond and the solvent comprising an alcohol are added.

When the iminium ion is combined with the compound comprising a non-aromatic C-C double bond, it is advantageous to control the temperature. Preferably, the step of combining the iminium ion with the compound comprising a non-aromatic C-C double bond is carried out at a reaction temperature in the range of -20°C to 30°C, more preferably -10°C to 20°C, still more preferably -5°C to 10°C. As will be understood by the skilled person, the minimum temperature should be higher than the freezing point of the components, such as the iminium salt and the compound comprising a non-aromatic C-C double bond.

The hydroaminomethylation reaction can be initiated e.g. by simply adding the compound comprising a non-aromatic C-C double bond to the iminium ion, or by adding the iminium ion to the compound comprising a non-aromatic C-C double bond and mixing the two.

If necessary, e.g. if a compound comprising a non-aromatic C-C double bond is used which is in a gaseous state under standard conditions (e.g. 25 °C, 100 kPa), the reaction can be carried out under increased pressure in a suitable apparatus.

Generally, no further components are required to carry out the reaction, and the solvent comprising an alcohol is used as a reaction medium.

The reaction of the compound comprising a non-aromatic C-C double bond with the iminium ion is typically carried out in the absence of a metal catalyst or a photocatalyst, preferably in the absence of both a metal catalyst and a photocatalyst. As will be understood by the skilled reader, such catalytically active components do not act as reatctants themselves, but promote the reaction, e.g. by reducing the required activation energy, or, especially in the case of a photocatalyst, by absorbing light and making the energy of the absorbed light available for the reaction that is to be catalyzed.

Moreover, the reaction of the compound comprising a non-aromatic C-C double bond with the iminium ion is also typically carried out in the absence of an acid. Preferably, the reaction of the compound comprising a non-aromatic C-C double bond with the iminium ion is carried out in the absence of a metal catalyst, a photocatalyst and an acid.

An acid as refered to in this context is defined as a proton donating acid having a pKₐ value at 25°C in water of 6 or less. Examples of the acid include a mineral acid (such as sulfuric acid) and/or a carboxylic acid including substituted carboxylic acids (such as acetic acid and trifluoroacetic acid).

The reaction temperature for the reaction between the compound comprising a non-aromatic C-C double bond with the reactive component can be suitably adjusted so that the reaction proceeds at an appropriate rate. In fact, the inventors have found that the reaction can be efficiently carried out at temperatures which are lower than those reported for known hydroaminomethylation reactions. For example, the reaction can be carried out at a reaction temperature in the range of 0°C to 150°C, preferably 20°C to 120°C, and more preferably 45°C to 90°C.

A suitable reaction time for the reaction between the compound comprising a non-aromatic C-C double bond with the reactive component is from 10 min to 72 h, preferably from 20 min to 20 h and more preferably from 30 min to 8 h.

Preferably, the iminium ion is used in the process according to the present invention in an amount of 1 molar equivalent or more, more preferably 1.5 molar equivalents or more, and still more preferably 3 molar equivalents or more, for each mole of non-aromatic C-C double bond that is subjected to the hydroaminomethylation reaction. Maximum amounts are not particularly limited. However, too high amounts may not be economic. Typically, amounts of 10 molar equivalents or less, preferably 5 molar equivalents or less are used.

Following the reaction of the compound comprising a non-aromatic C-C double bond with the iminium ion, the process in accordance with the invention allows the structure of the resulting amine to be varied via a further reaction of the product which is obtained from the reaction of the compound comprising a non-aromatic C-C double bond with the iminium ion.

Without wishing to be bound by theory, it is assumed that the iminium ion acts as a reactive species in a hydroaminomethylation reaction. As illustrated by the reaction scheme 2 below, the iminium ion is added as an electrophile to the non-aromatic double bond (e.g. in compound (2)), followed by a hydride shift from a substituent that was originally bound to the nitrogen atom of the iminium ion. This sequence as shown in reaction scheme 2 is intended as an illustration of the possible mechanism of the reaction underlying the claimed process, and not as a limitation thereof. The groups R, R^{A} and R^{B} represent any desired atoms or groups. It should be understood that the iminium ion and the compound comprising a non-aromatic double bond used in the present invention are not limited to the exemplified structures (1) and (2).

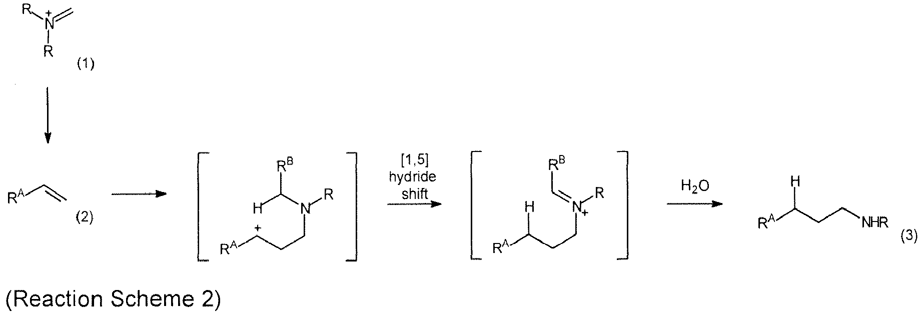

As a result of the hydroaminomethylation reaction, the C-C double bond is thus converted into a single bond (e.g. in compound (3)).

The addition of water to the reaction mixture resulting from the reaction of the compound comprising a non-aromatic C-C double bond with the iminium ion thus results in the formation of a compound containing a secondary amino group, as illustrated in reaction scheme 2. For a compound comprising a C-C double bond used as a starting material, this secondary amino group is bound via a methylene group to one of the carbon atoms of the former C-C double bond that has been converted into a single bond by the hydroaminomethylation reaction.

Thus, in one preferred embodiment, the process in accordance with the invention further comprises a step wherein water is added to the reaction mixture resulting from the step of reacting a compound comprising a non-aromatic C-C double bond with the reactive component.

For example, the water may be conveniently added in the form of an aqueous solution of a base, i.e. a solution of a base in water. Thus, the addition of the water also accomplishes a work-up of the reaction mixture before the product is isolated. For example, the solution of the base can have a concentration of the base between 0.1 mol/l and 4 mol/l, preferably between 0.5 mol/l and 2 mol/l. Examples of the base comprise ammonia, a metal carbonate, a metal hydrogencarbonate and a metal hydroxide. The metal is preferably selected from sodium, potassium, calcium, lithium and magnesium. Preferred examples of the base are sodium hydroxide, potassium hydroxide, calcium carbonate and calcium hydrogencarbonate.

In another preferred embodiment, the process in accordance with the invention further comprises a step wherein an organic nucleophilic reactant is added to the reaction mixture resulting from the step of reacting a compound comprising a non-aromatic C-C double bond with the iminium ion.

The addition of such an organic nucleophilic reactant allows a tertiary amine to be provided. In this regard, it is assumed that the nucleophilic organic reactant can undergo a direct reaction with an intermediate product which contains an iminium group as illustrated in reaction scheme 2 above. The resulting amine contains a tertiary amino group. For a compound comprising a C-C double bond used as a starting material, also this tertiary amino group is bound via a methylene group to one of the carbon atoms of the former C-C double bond that has been converted into a single bond by the hydroaminomethylation reaction.

The present inventors surprisingly found that the mild conditions of the reported process allow a reaction of the intermediate product with negatively charged, basic hydrides or alkyl-metal reagents in order to obtain tertiary amines in high yields. Examples of suitable organic nucleophilic reactants include borohydrides and organometal compounds. Borohydrides may be represented by the general formula MBH₄₋ₙXₙ⁻, wherein M is a monovalent metal, such as Li, Na and K; n is 0 to 3; and X is ethyl, methyl, cyano, and acetate. Examples of borohydrides include LiBH₄, NaBH₄, KBH₄, NaBH(OAc)₃ and LiBH(Et)₃. Organometal compounds are preferably alkyl metal compounds, such as alkyl lithium compounds, alkyl magnesium compounds, alkyl cuprate compounds or alkyl zinc compounds, more preferably alkyl zinc compounds, such as dimethylzinc or diethylzinc.

Preferably, the organic nucleophilic reactant is added in an amount of 4 molar equivalents or more, more preferably 8 molar equivalents or more, and still more preferably 12 molar equivalents or more, for each mole of non-aromatic C-C double bond that is subjected to the hydroaminomethylation reaction. Maximum amounts are not particularly limited. However, too high amounts may not be economic.

Following the reaction with the nucleophilic organic reactant, a solution of a base in water can be added before the product is isolated.

In line with the above, a preferred embodiment of the process in accordance with the present invention can be defined as a process for producing an amine via a hydroaminomethylation reaction of a non-aromatic C-C double bond, said process comprising
a step of reacting a compound comprising a non-aromatic C-C double bond with an iminium ion in a solvent comprising an alcohol, and
a step of adding water or a nucleophilic organic reactant to the reaction mixture resulting from the step of reacting a compound comprising a non-aromatic C-C double bond with the iminium ion,
wherein the iminium ion is represented by the following formula (I): 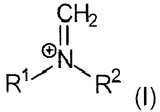 wherein R¹ and R² are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted; and R¹ and R² may be bonded to each other to form a ring together with the nitrogen atom to which they are bound; and wherein R¹ and R² are not both hydrogen and at least one of R¹ and R² carries a hydrogen atom at a carbon atom in α-position to the nitrogen atom of the iminium ion.

In line with the above, in accordance with a further preferred embodiment, the process for producing an amine via a hydroaminomethylation reaction of a non-aromatic C-C double bond, may thus be a process comprising
a step of reacting a compound comprising a non-aromatic C-C double bond with an iminium ion in a solvent comprising or consisting of a fluorinated alcohol, and
a step of adding water or a nucleophilic organic reactant to the reaction mixture resulting from the step of reacting a compound comprising a non-aromatic C-C double bond with the iminium ion,
wherein the iminium ion is dimethylmethylideneammonium and wherein the fluorinated alcohol is preferably selected from the group consisting of 1-fluoroethanol; 2-fluoroethanol; trifluoroethanol; hexafluoroisopropanol; 2- fluorophenol; 3- fluorophenol; 4-fluorophenol and combinations thereof, more preferably hexafluoroisopropanol, trifluoroethanol, and combinations thereof.

Moreover, conventional steps in amine synthesis reactions can be comprised in the process according to the present invention, such as the protection or the deprotection of an amino group. For example, if an iminium ion is used in the process in accordance with the invention wherein R¹ and/or R² in formula (I) are a protecting group, such as a benzyl group, an allyl group or a Boc-group, the process of the present invention may further comprise a step of a deprotection of the amino group following the reaction of a compound comprising a non-aromatic C-C double bond with the iminium ion. The deprotection reaction is not particularly limited and typical deprotection reactions are known in the art.

Alternatively, where desired, it is also possible to protect an amine resulting from the hydroaminomethylation reaction by introducing a protective group for the amine, e,g, a benzyl group, an allyl group or a Boc-group.

Finally, it will be understood that also typical steps for the isolation and/or the purification of a desired product can form part of the process in accordance with the invention, so that the desired product or products can be obtained in high yields and a good quality.

Important aspects of the present invention are summarised in the following items. It will be understood that those items referring back to a preceeding item reflect preferred embodiments of the invention.
1. A process for producing an amine via a hydroaminomethylation reaction of a non-aromatic C-C double bond, said process comprising
   a step of reacting a compound comprising a non-aromatic C-C double bond with an iminium ion in a solvent comprising an alcohol,
   wherein the iminium ion is represented by the following formula (I): 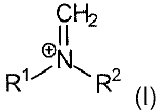 wherein,
   R¹ and R² are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted;
   and R¹ and R² may be bonded to each other to form a ring together with the nitrogen atom to which they are bound;
   and wherein R¹ and R² are not both hydrogen and at least one of R¹ and R² carries a hydrogen atom at a carbon atom in α-position to the nitrogen atom of the iminium ion.
2. The process according to item 1, wherein the solvent consists of an alcohol.
3. The process according to item 1 or 2, wherein the alcohol is a halogenated alcohol.
4. The process according any of items 1 to 3, wherein the alcohol is a fluorinated alcohol.
5. The process according to item 4, wherein the fluorinated alcohol is selected from the group consisting of 1-fluoroethanol; 2-fluoroethanol; trifluoroethanol; 3,3,3-trifluoro-1-propanol; 1,1,1-trifluoro-2-propanol; 2,2,3,3,3-pentafluoro-1-propanol; hexafluoroisopropanol; 1,1,1-trifluoro-2-butanol; 4,4,4-trifluoro-1-butanol; 1,1,1-trifluoro-3-methyl-2-butanol; 4,4,5,5,5-pentafluoro-1-pentanol; 3-fluorophenethyl alcohol; 2,4,5-trifluorobenzyl alcohol; 2-fluorophenol; 3- fluorophenol; 4-fluorophenol; and combinations thereof;
   more preferably selected from the group consisting of 1-fluoroethanol; 2-fluoroethanol; trifluoroethanol; hexafluoroisopropanol; 2- fluorophenol; 3- fluorophenol; 4-fluorophenol; and combinations thereof.
6. The process according to item 4 or 5, wherein the fluorinated alcohol is selected from the group consisting of hexafluoroisopropanol, trifluoroethanol, and combinations thereof.
7. The process according to any of items 1 to 6, which comprises a step of combining the iminium ion in the form of an iminium salt with the compound comprising a non-aromatic C-C double bond in the solvent comprising the alcohol.
8. The process according to item 7, wherein said step of combining the iminium ion with the compound comprising a non-aromatic C-C double bond is carried out at a reaction temperature in the range of -20°C to 30°C, more preferably -10°C to 20°C, still more preferably -5°C to 10°C.
9. The process according to any of items 1 to 8, wherein, in formula (I),
   R¹ and R² are independently selected from the group consisting of a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted;
   and R¹ and R² may be bonded to each other to form a ring together with the nitrogen atom to which they are bound.
10. The process according to any of items 1 to 9, wherein R¹ and R² are independently selected from the group consisting of methyl, ethyl, propyl, isopropyl, butyl, isobutyl, benzyl and allyl.
11. The process according to any of items 1 to 10, wherein R¹ and R² are identical.
12. The process according to any of items 1 to 11, wherein the iminium ion is dimethylmethylideneammonium.
13. The process according to any of items 7 to 12, wherein the iminium salt comprises an anion selected from iodide, bromide, chloride, nitrate, triflate and trifluoroacetate.
14. The process according to any of items 7 to 13, wherein the iminium salt is dimethylmethylideneammonium iodide (Eschenmoser's salt).
15. The process according to any of items 1 to 14, wherein the iminium ion is used in an amount of 1 molar equivalent or more, more preferably 1.5 molar equivalents or more, and still more preferably 3 molar equivalents or more, for each mole of non-aromatic C-C double bond that is subjected to the hydroaminomethylation reaction.
16. The process according to any of items 1 to 15, wherein the compound comprising a non-aromatic C-C double bond is represented by the following formula (II): 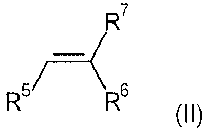 wherein R⁵, R⁶ and R⁷ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, and wherein R⁵ and R⁶ may be bonded to each other to form a ring together with the carbon atoms to which they are bound.
17. The process according to item 16, wherein R⁵, R⁶ and R⁷ are independently selected from the group consisting of hydrogen, a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted, and R⁵ and R⁶ may be bonded to each other to form a ring together with the carbon atoms to which they are bound.
18. The process according to item 16 or 17, wherein
   R⁵ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic hydrocarbon group which may be substituted; and either
   (i) R⁶ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic group which may be substituted; R⁵ and R⁶ may be bonded to each other to form a ring together with the carbon atoms to which they are bound; and R⁷ is selected from the group consisting of hydrogen and methyl, and is more preferably hydrogen; or
   (ii) R⁷ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic group which may be substituted; and R⁶ is selected from the group consisting of hydrogen and methyl, and is more preferably hydrogen.
19. The process according to any of items 1 to 18, wherein said step of reacting the compound comprising a non-aromatic C-C double bond with the iminium ion is carried out in the absence of a metal catalyst.
20. The process according to any of items 1 to 19, wherein said step of reacting the compound comprising a non-aromatic C-C double bond with the iminium ion is carried out in the absence of a photocatalyst.
21. The process according to any of items 1 to 20, wherein said step of reacting the compound comprising a non-aromatic C-C double bond with the iminium ion is carried out in the absence of an acid.
22. The process according to any of items 1 to 21, wherein said step of reacting the compound comprising a non-aromatic C-C double bond with the iminium ion is carried out at a reaction temperature in the range of 0°C to 150°C, more preferably 20 to 120 °C, still more preferably 45 to 90°C
23. The process according to any of items 1 to 22, wherein the reaction time for the reaction of the compound comprising a non-aromatic C-C double bond with the iminium ion ranges from 10 min to 72 h, more preferably from 20 min to 20 h, still more preferably from 30 min to 8 h.
24. The process according to any of items 1 to 23, which further comprises a step wherein water is added to the reaction mixture resulting from the step of reacting a compound comprising a non-aromatic C-C double bond with the iminium ion, and wherein the water is preferably added to the reaction mixture in the form of an aqueous solution of a base.
25. The process according to any of items 1 to 23, which further comprises a step wherein an organic nucleophilic reactant is added to the reaction mixture resulting from the step of reacting a compound comprising a non-aromatic C-C double bond with the iminium ion.
26. The process according to item 25, wherein the organic nucleophilic reactant is selected from borohydrides and organometal compounds.
27. The process according to item 25 or 26, wherein the organic nucleophilic reactant is added in an amount of 4 molar equivalents or more, more preferably 8 molar equivalents or more, and still more preferably 12 molar equivalents or more, for each mole of non-aromatic C-C double bond that is subjected to the hydroaminomethylation reaction.

### Examples

### General Information

All glassware was oven dried at 100 °C before use. All solvents were distilled from appropriate drying agents prior to use. All reagents were used as received from commercial suppliers unless otherwise stated. Neat infrared spectra were recorded using a Perkin-Elmer Spectrum 100 FT-IR spectrometer. Wavenumbers (v = 1/λ) are reported in cm⁻¹. Mass spectra were obtained using a Finnigan MAT 8200 or (70 eV) or an Agilent 5973 (70 eV) spectrometer, using electrospray ionization (ESI) All ¹H NMR and ¹³C NMR experiments were recorded using Bruker AV-400, AV-600 and AV-700 spectrometers at 300 K. Chemical shifts (δ) are quoted in ppm and coupling constants (J) are quoted in Hz. The 7.26 ppm resonance of residual CHCl3 for proton spectra and 77.16 ppm resonance for carbon spectra were used as internal references. 1H NMR splitting patterns were designated as singlet (s), doublet (d), triplet (t), quartet (q) or combinations thereof, as well as broad (br). Splitting patterns that could not be interpreted were designated as multiplet (m). Reaction progress was monitored by thin layer chromatography (TLC) performed on aluminum plates coated with kieselgel F254 with 0.2 mm thickness. Visualization was achieved by a combination of ultraviolet light (254 nm) and acidic potassium permanganate. Flash column chromatography was performed using silica gel 60 (230-400 mesh, Merck and co.).

### General procedure for synthesis of secondary amines 4 (Examples 1 to 4; Note: Example 4 uses 2,2,2-trifluoroethanol instead of HFIP as solvent)

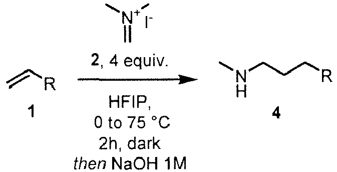

A round-bottom flask covered from light was charged with *N,N*-Dimethylmethyleneiminium iodide (Eschenmoser's iodide, 4 equiv.) and a magnetic stir-bar under argon-atmosphere was cooled to 0 °C. After this, the alkene (1 equivalent) and 1,1,1,3,3,3-hexafluoroisopropanol (HFIP, 0.6 M of alkene in HFIP) were added. After completed addition of the solvent, the flask was sealed and placed in an oil bath at 75 °C. The reaction was vigorously stirred at this temperature for 2 h, after which it was allowed to cool to room temperature. Subsequently, aqueous sodium hydroxide (1 M) was added until the reaction mixture reaches pH 12. The resulting biphasic mixture was separated and the aqueous phase was extracted with dichloromethane (3 x 200 mL/mmol). The combined organic phases were then dried over anhydrous potassium carbonate and filtered. The filtrate was concentrated under reduced pressure to afford the crude product, which was purified by flash column chromatography on silica gel (dichloromethane/MeOH/NH4OH 19:1:0.15) to afford the analytically pure desired product.

### Example 1: N-methyl-3-phenylpropan-1-amine 4a

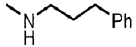

100% NMR yield; ¹H NMR (400 MHz, CDCl₃) δ 7.30-7.25 (m, 2H), 7.22-7.15 (m, 3H), 2.66 (t, *J* = 7.8 Hz, 2H), 2.61 (t, *J* = 7.2 Hz, 2H), 2.43 (s, 3H), 1.86-1.77 (m, 2H), 1.02 (br s, 1H); ¹³C NMR (100 MHz, CDCl₃) δ 142.4, 128.5 (2C), 128.4 (2C), 125.9, 51.8, 36.7, 33.8, 31.7; IR (neat) vmax: 3026, 2932, 2859, 1541, 1493, 1454, 1383, 1306, 1252, 748, 700; HRMS (ESI+): exact mass calculated for [M+H]+ (C10H16N) requires *m*/*z* 150.1277, found *mlz* 150.1274.

### Example 2: N-methyldodecan-1-amine 4b

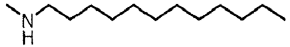

75% yield; ¹H NMR (400 MHz, CDCl₃) δ 2.54 (t, *J* = 7.3 Hz, 2H), 2.42 (s, 3H), 1.46 (app quin, *J* = 7.1 Hz, 2H), 1.32-1.21 (m, 18H), 1.10 (br s, 1H), 0.87 (t, *J* = 7.0 Hz, 3H); ¹³C NMR (100 MHz, CDCl₃) δ 52.4, 36.8, 32.1, 30.1, 29.8, 29.8, 29.7 (2C), 29.7, 29.5, 27.5, 22.8, 14.3; IR (neat) vₘₐₓ: 2955, 2921, 2852, 1465, 1380, 1308, 722; HRMS (ESI+): exact mass calculated for [M+H]⁺ (C₁₃H₃₀N) requires *mlz* 200.2373, found *m*/*z* 200.2367.

### Example 3: N-methyl-5-phenylpentan-1-amine 4c

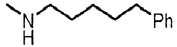

79% yield; ¹H NMR (400 MHz, CDCl₃) δ 7.30-7.24 (m, 2H), 7.21-7.15 (m, 3H), 2.62 (t, *J* = 7.8 Hz, 2H), 2.56 (t, *J* = 7.2 Hz, 2H), 2.42 (s, 3H), 1.69- 1.60 (m, 2H), 1.56-1.47 (m, 2H), 1.42-1.33 (m, 2H), 0.91 (br s, 1H); ¹³C NMR (100 MHz, CDCl₃) δ 142.8, 128.5 (2C), 128.4 (2C), 125.7, 52.3, 36.7, 36.0, 31.5, 30.0, 27.1; IR (neat) vmax: 3025, 2927, 2854, 2788, 1454, 1381, 1308, 811, 744, 698; HRMS (ESI+): exact mass calculated for [M+H]+ (C12H20N) requires *mlz* 178.1590, found *m*/*z* 178.1590.

### General procedure for synthesis of tertiary amines 5 (Examples 5 to 7)

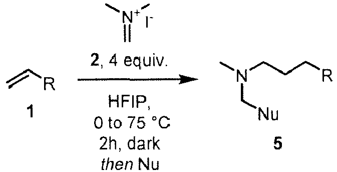

### Example 5: N,N-dimethyl-3-phenylpropan-1-amine 5a

A round-bottom flask covered from light was charged with *N,N*-Dimethylmethyleneiminium iodide (Eschenmoser's iodide, 4 equiv.) and a magnetic stir-bar under argon-atmosphere was cooled to 0 °C. After this, styrene (1 equivalent) and 1,1,1,3,3,3-hexafluoroisopropanol (HFIP, 0.6 M of alkene in HFIP) were added. After completed addition of the solvent, the flask was sealed and placed in an oil bath at 75 °C. The reaction was vigorously stirred at this temperature for 2 h, after which it was allowed to cool to room temperature. Subsequently, NaBH(OAc)₃ was added (15 equivalents with respect to the alkene) followed from methanol (10 ml/mmol). The reaction was stirred at room temperature for 6 hours; then aqueous sodium hydroxide (1 M) was added until the reaction mixture reached pH 12. The resulting biphasic mixture was separated and the aqueous phase was extracted with dichloromethane (3 x 200 mL/mmol). The combined organic phases were then dried over anhydrous potassium carbonate and filtered. The filtrate was concentrated under reduced pressure to afford the crude product, which was purified by flash column chromatography on silica gel (dichloromethane/MeOH/NH4OH 19:1:0.15) to afford the analytically pure desired product.

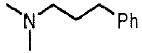

74% yield; ¹H NMR (400 MHz, CDCl₃) δ 7.30 - 7.25 (m, 2H), 7.20 - 7.16 (m, 3H), 2.67 - 2.63 (m, 2H), 2.36 - 2.27 (m, 2H), 2.27 (s, 6H), 1.82 (dt, J = 15.2, 7.6 Hz, 2H); ¹³C NMR (100 MHz, CDCl₃) δ 142.2, 128.5 (2C), 128.5 (2C), 125.9, 59.3, 45.5 (2C), 33.7, 29.3; IR (neat) vₘₐₓ: 3403, 3061, 3026, 2922, 2852, 2781, 1667, 1602, 1495, 1454, 1377, 1030; HRMS (ESI+): exact mass calculated for [M+H]⁺ (C₁₁H₁₈N) requires *mlz* 164.1434, found *m*/*z* 164.1438.

### Example 6: N-ethyl-N-methyl-3-phenylpropan-1-amine 5b

A round-bottom flask covered from light was charged with *N,N*-Dimethylmethyleneiminium iodide (Eschenmoser's iodide, 4 equiv.) and a magnetic stir-bar under argon-atmosphere was cooled to 0 °C. After this, styrene (1 equivalent) and 1,1,1,3,3,3-hexafluoroisopropanol (HFIP, 0.6 M of alkene in HFIP) were added. After completed addition of the solvent, the flask was sealed and placed in an oil bath at 75 °C. The reaction was vigorously stirred at this temperature for 2 h, after which it was allowed to cool to room temperature. Subsequently, dimethylzinc was added (15 equivalents with respect to the alkene, 1 mol/L solution in heptane). The reaction was stirred at room temperature for 5 hours. Water was slowly added to quench the reaction, then aqueous sodium hydroxide (1 M) was added until the reaction mixture reached pH 12. The resulting biphasic mixture was separated and the aqueous phase was extracted with dichloromethane (3 x 200 mL/mmol). The combined organic phases were then dried over anhydrous potassium carbonate and filtered. The filtrate was concentrated under reduced pressure to afford the crude product, which was purified by flash column chromatography on silica gel (dichloromethane/MeOH/NH4OH 19:1:0.15) to afford the analytically pure desired product.

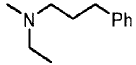

66% yield; ¹H NMR (400 MHz, CDCl₃) δ 7.30 - 7.26 (m, 2H), 7.19 (d, *J* = 7.4 Hz, 3H), 2.64 (t, *J* = 7.8 Hz, 2H), 2.50 - 2.47 (m, 4H), 2.26 (s, 3H), 1.86 (dd, *J* = 15.3, 7.8 Hz, 2H), 1.08 (t, *J* = 7.2 Hz, 3H); ¹³C NMR (100 MHz, CDCl₃) δ 142.2, 128.5 (2C), 128.5 (2C), 125.9, 56.8, 51.5, 41.6, 33.9, 28.9, 12.1; IR (neat) vₘₐₓ: 3433, 3351, 2943, 2932, 2361, 1495, 1455, 750, 701; HRMS (ESI+): exact mass calculated for [M+H]⁺ (C₁₂H₂₀N) requires *m*/*z* 178.1590, found *m*/*z* 178.1592.

### Example 7: N-methyl-3-phenyl-N-propylpropan-1-amine 5c

A round-bottom flask covered from light was charged with *N,N*-Dimethylmethyleneiminium iodide (Eschenmoser's iodide, 4 equiv.) and a magnetic stir-bar under argon-atmosphere was cooled to 0 °C. After this, styrene (1 equivalent) and 1,1,1,3,3,3-hexafluoroisopropanol (HFIP, 0.6 M of alkene in HFIP) were added. After completed addition of the solvent, the flask was sealed and placed in an oil bath at 75 °C. The reaction was vigorously stirred at this temperature for 2 h, after which it was allowed to cool to room temperature. Subsequently, diethylzinc was added (15 equivalents with respect to the alkene, 1 mol/L solution in hexanes). The reaction was stirred at room temperature for 6 hours. Water was slowly added to quench the reaction, then aqueous sodium hydroxide (1 M) was added until the reaction mixture reached pH 12. The resulting biphasic mixture was separated and the aqueous phase was extracted with c (3 x 200 mL/mmol). The combined organic phases were then dried over anhydrous potassium carbonate and filtered. The filtrate was concentrated under reduced pressure to afford the crude product, which was purified by flash column chromatography on silica gel (dichloromethane/MeOH/NH4OH 19:1:0.15) to afford the analytically pure desired product.

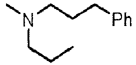

63% yield; ¹H NMR (400 MHz, CDCl₃) δ 7.30 - 7.26 (m, 21H), 7.20 - 7.16 (m, 3H), 2.65 - 2.61 (m, 2H), 2.39 - 2.35 (m, 2H), 2.29 (dd, *J* = 8.6, 6.7 Hz, 2H), 2.22 (s, 3H), 1.80 (ddd, *J* = 15.1, 8.5, 6.8 Hz, 21H), 1.52 - 1.43 (m, 2H), 0.89 (t, *J* = 7.4 Hz, 3H); ¹³C NMR (100 MHz, CDCl₃) δ 142.6, 128.5, 128.4, 125.8, 60.0, 57.5, 42.4, 33.9, 29.2, 20.6, 12.1; ; IR (neat) vₘₐₓ: 3433, 3352, 3207, 2954, 2922, 2852,1658, 1633, 1467, 1422; HRMS (ESI+): exact mass calculated for [M+H]⁺ (C₁₃H₂₂N) requires *mlz* 192.1747, found *m*/*z* 192.1749.

### General procedure for Comparative Examples 1 to 3

A round-bottom flask covered from light was charged with N,N-Dimethylmethyleneiminium salt (4 equiv.) and a magnetic stir-bar under argon-atmosphere was cooled to 0 °C. After this, the alkene (1 equivalent) and solvent (as indicated in the table) were added. After completed addition of the solvent, the flask was sealed and placed in an oil bath at the indicated temperature. The reaction was vigorously stirred at this temperature for the indicated time, after which it was allowed to cool to room temperature. Subsequently, aqueous sodium hydroxide (1 M) was added until the reaction mixture reaches pH 12. The resulting biphasic mixture was separated and the aqueous phase was extracted with dichloromethane (3 x 200 mL/mmol). The combined organic phases were then dried over anhydrous potassium carbonate and filtered. The filtrate was concentrated under reduced pressure to afford the crude product, which was purified by flash column chromatography on silica gel (dichloromethane/MeOH/NH4OH 19:1:0.15) to afford the analytically pure desired product.

**Table 1**

| | | | | | | |
|---|---|---|---|---|---|---|
| 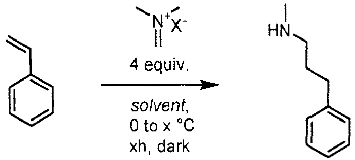 | | | | | | |

| | *X* | *solvent* | *Concentration of alkene* | *temperature* | *Time (h)* | *NMR yield* |
|---|---|---|---|---|---|---|
| Comparative Example 1 | Cl⁻ | AcOH | 0.6M | 23 °C | 16 | 0% |
| Comparative Example 2 | Cl⁻ | AcOH | 0.6M | 75 °C | 16 | 33% |
| Comparative Example 3 | Cl⁻ | MeCN | 0.2M | 75 °C | 16 | 35% |
| Example 1 | I⁻ | HFIP | 0.6M | 75°C | 2 | 100% |
| Example 4 | I⁻ | 2,2,2-trifluoroethanol | 06M | 75 °C | 2 | 75% |

As can be taken from Table 1, the hydroaminomethylation reaction according to the present invention resulted in high yields and high selectivity (Examples 1 and 4). In contrast, the hydroaminomethylation using dimethylmethylideneammonium in acetic acid or acetonitrile resulted in low yields (Comparative Examples 1 to 3).

## Claims

1. A process for producing an amine via a hydroaminomethylation reaction of a non-aromatic C-C double bond, said process comprising
a step of reacting a compound comprising a non-aromatic C-C double bond with an iminium ion in a solvent comprising an alcohol,
wherein the iminium ion is represented by the following formula (I): 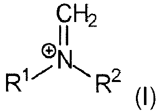 wherein,
R¹ and R² are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, such as an aralkyl group which may be substituted;
and R¹ and R² may be bonded to each other to form a ring together with the nitrogen atom to which they are bound;
and wherein R¹ and R² are not both hydrogen and at least one of R¹ and R² carries a hydrogen atom at a carbon atom in α-position to the nitrogen atom of the iminium ion.

2. The process according to claim 1, wherein the solvent consists of an alcohol.

3. The process according to claim 1 or 2, wherein the alcohol is a halogenated alcohol.

4. The process according any of claims 1 to 3, wherein the alcohol is a fluorinated alcohol.

5. The process according to claim 4, wherein the fluorinated alcohol is selected from the group consisting of 1-fluoroethanol; 2-fluoroethanol; trifluoroethanol; 3,3,3-trifluoro-1-propanol; 1,1,1-trifluoro-2-propanol; 2,2,3,3,3-pentafluoro-1-propanol; hexafluoroisopropanol; 1,1,1-trifluoro-2-butanol; 4,4,4-trifluoro-1-butanol; 1,1,1-trifluoro-3-methyl-2-butanol; 4,4,5,5,5-pentafluoro-1-pentanol; 3-fluorophenethyl alcohol; 2,4,5-trifluorobenzyl alcohol; 2-fluorophenol; 3- fluorophenol; 4-fluorophenol; and combinations thereof;
more preferably selected from the group consisting of 1-fluoroethanol; 2-fluoroethanol; trifluoroethanol; hexafluoroisopropanol; 2- fluorophenol; 3- fluorophenol; 4-fluorophenol; and combinations thereof.

6. The process according to any of claims 1 to 5, wherein, in formula (I),
R¹ and R² are independently selected from the group consisting of a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted;
and R¹ and R² may be bonded to each other to form a ring together with the nitrogen atom to which they are bound.

7. The process according to any of claims 1 to 6, wherein R¹ and R² are independently selected from the group consisting of methyl, ethyl, propyl, isopropyl, butyl, isobutyl, benzyl and allyl.

8. The process according to any of claims 1 to 7, wherein the iminium ion is dimethylmethylideneammonium.

9. The process according to any of claims 1 to 8, wherein the iminium ion is in the form of an iminium salt and the iminium salt comprises an anion selected from iodide, bromide, chloride, nitrate, triflate and trifluoroacetate.

10. The process according to any of claims 1 to 9, wherein the iminium ion is used in an amount of 1 molar equivalent or more, more preferably 1.5 molar equivalents or more, and still more preferably 3 molar equivalents or more, for each mole of non-aromatic C-C double bond that is subjected to the hydroaminomethylation reaction.

11. The process according to any of claims 1 to 10, wherein the compound comprising a non-aromatic C-C double bond is represented by the following formula (II): 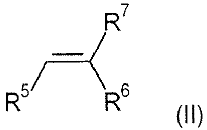 wherein R⁵, R⁶ and R⁷ are independently hydrogen or selected from the group consisting of an aliphatic hydrocarbon group which may be substituted, an aromatic hydrocarbon group which may be substituted, an aliphatic heterocyclic group which may be substituted, an aromatic heterocyclic group which may be substituted and of combinations thereof, and wherein R⁵ and R⁶ may be bonded to each other to form a ring together with the carbon atoms to which they are bound.

12. The process according to claim 11, wherein R⁵, R⁶ and R⁷ are independently selected from the group consisting of hydrogen, a linear or branched alkyl group which may be substituted, a cycloalkyl group which may be substituted, a heterocycloalkyl group which may be substituted, a linear or branched alkenyl group which may be substituted, a cycloalkenyl group which may be substituted, a linear or branched alkynyl group which may be substituted, an aromatic hydrocarbon group which may be substituted, an aralkyl group which may be substituted and a heteroaromatic group which may be substituted, and R⁵ and R⁶ may be bonded to each other to form a ring together with the carbon atoms to which they are bound.

13. The process according to claim 11 or 12, wherein
R⁵ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic hydrocarbon group which may be substituted; and either
(i) R⁶ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic group which may be substituted; R⁵ and R⁶ may be bonded to each other to form a ring together with the carbon atoms to which they are bound; and R⁷ is selected from the group consisting of hydrogen and methyl, and is more preferably hydrogen; or
(ii) R⁷ is selected from the group consisting of hydrogen, an alkyl group which may be substituted, a cycloalkyl group which may be substituted, a heterocycloalkyl group which may be substituted, an alkenyl group which may be substituted, a cycloalkenyl group which may be substituted, an alkynyl group which may be substituted, an aromatic hydrocarbon group which may be substituted, an aralkyl group which may be substituted and a heteroaromatic group which may be substituted; and R⁶ is selected from the group consisting of hydrogen and methyl, and is more preferably hydrogen.

14. The process according to any of claims 1 to 13, wherein said step of reacting the comppund comprising a non-aromatic C-C double bond with the iminium ion is carried out in the absence of a metal catalyst.

15. The process according to any of claims 1 to 14, wherein said step of reacting the compound comprising a non-aromatic C-C double bond with the iminium ion is carried out in the absence of a photocatalyst.

16. The process according to any of claims 1 to 15, wherein said step of reacting the compound comprising a non-aromatic C-C double bond with the iminium ion is carried out in the absence of an acid.

17. The process according to any of claims 1 to 16, wherein said step of reacting the compound comprising a non-aromatic C-C double bond with the iminium ion is carried out at a reaction temperature in the range of 0°C to 150°C, more preferably 20 to 120 °C, still more preferably 45 to 90°C

18. The process according to any of claims 1 to 17, wherein the reaction time for the reaction of the compound comprising a non-aromatic C-C double bond with the iminium ion ranges from 10 min to 72 h, more preferably from 20 min to 20 h, still more preferably from 30 min to 8 h.

19. The process according to any of claims 1 to 18, which further comprises a step wherein water is added to the reaction mixture resulting from the step of reacting a compound comprising a non-aromatic C-C double bond with the iminium ion, and wherein the water is preferably added to the reaction mixture in the form of an aqueous solution of a base.

20. The process according to any of claims 1 to 18, which further comprises a step wherein an organic nucleophilic reactant is added to the reaction mixture resulting from the step of reacting a compound comprising a non-aromatic C-C double bond with the iminium ion.
